# EUROPÄISCHE PATENTSCHRIFT

(11) **EP 0 628 543 B1**
(45) Veröffentlichungstag und Bekanntmachung des Hinweises auf die Patenterteilung: **16.06.1999**
(21) Anmeldenummer: 94108280.2
(22) Anmeldetag: 30.05.1994
(51) Int. Cl.: C07C 279/22, C07D 233/61, C07C 317/48, A61K 31/155, A61K 31/415, A61K 31/165

(54) **Substituierte Benzoylguanidine, Verfahren zu ihrer Herstellung, ihre Verwendung als Medikament oder Diagnostikum sowie sie enthaltendes Medikament**
Substituted benzoylguanidines, process for their preparation, their use as medicament or diagnostic agent, as well as a medicament containing them
Benzoylguanidines substituées, procédé pour leur préparation, leur utilisation comme médicament ou agent diagnostique, ainsi que médicament les contenant

(30) Priorität: 05.06.1993 DE 4318756
(43) Veröffentlichungstag der Anmeldung: 14.12.1994
(73) Patentinhaber: HOECHST AKTIENGESELLSCHAFT, 65929 Frankfurt am Main (DE)
(72) Erfinder: Schwark, Jan-Robert, Dr., D-65929 Frankfurt (DE); Kleemann, Heinz-Werner, Dr., D-61350 Bad Homburg (DE); Lang, Hans-Jochen, Dr., D-65719 Hofheim/Taunus (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- EP-A- 0 556 673
- EP-A- 0 556 674
- US-A- 3 780 027

## Beschreibung

Die Erfindung betrifft Benzoylguanidine der Formel I 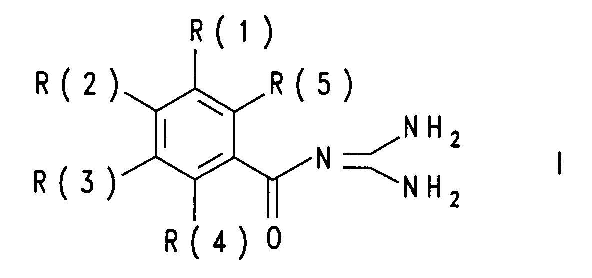 worin bedeuten:
R(1) R(6)-CO oder R(7)R(8)N-CO-;
   R(6) (C₁-C₈)-Alkyl, (C₁-C₈)-Perfluoralkyl, (C₃-C₈)-Alkenyl oder -CₙH₂ₙ-R(9);
      n Null, 1, 2, 3 oder 4;
      R(9) (C₃-C₈)-Cycloalkyl, Phenyl, Biphenylyl oder Naphthyl, wobei die Aromaten nicht substituiert oder substituiert sind mit 1 - 3 Substituenten aus der Gruppe bestehend aus F, Cl, CF₃, Methyl, Methoxy und NR(10)R(11);
         R(10) und R(11)
            H, (C₁-C₄)-Alkyl oder (C₁-C₄)-Perfluoralkyl;
   R(7) H, (C₁-C₄)-Alkyl oder CF₃;
   R(8) H, (C₁-C₄)-Alkyl oder CF₃;
R(2) wie R(1) definiert, oder H, F, Cl, Br, I, CN, (C₁-C₈)-Alkyl, (C₁-C₈)-Perfluoralkyl, (C₃-C₈)-Alkenyl oder -CₙH₂ₙR(15);
   n Null, 1, 2, 3, 4;
   R(15) (C₃-C₈)-Cycloalkyl, Phenyl, Biphenylyl oder Naphthyl,
      wobei die Aromaten nicht substituiert oder substituiert sind mit 1 - 3 Substituenten aus der Gruppe bestehend aus F, Cl, CF₃, Methyl, Methoxy und NR(16)R(17);
      R(16) und R(17)
         H, (C₁-C₄)-Alkyl oder (C₁-C₄)-Perfluoralkyl;
   oder
R(2) (C₁-C₉)-Heteroaryl,
   das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
   oder
R(2) -OR(18);
   R(18)-CₐH₂ₐ-(C₁-C₉)-Heteroaryl
      das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
      a Null, 1 oder 2;
   oder
R(2) R(21)-SOₘ;
   m 1 oder 2;
   R(21) (C₁-C₈)-Alkyl, (C₁-C₈)-Perfluoralkyl, (C₃-C₈)-Alkenyl oder -CₙH₂ₙ-R(24);
      n Null, 1, 2, 3 oder 4;
      R(24) (C₃-C₈)-Cycloalkyl, Phenyl, Biphenylyl oder Naphthyl,
         wobei die Aromaten nicht substituiert oder substituiert sind mit 1 - 3 Substituenten aus der Gruppe bestehend aus F, Cl, CF₃, Methyl, Methoxy und NR(27)R(28);
         R(27) und R(28)
            H, (C₁-C₄)-Alkyl oder (C₁-C₄)-Perfluoralkyl;
   oder
R(2) R(33)X;
   X Sauerstoff, S oder NR(34);
      R(33) (C₁-C₈)-Alkyl, (C₃-C₈)-Alkenyl, (CH₂)_{b}C_{d}F_{2d+1} oder -CₙH₂ₙ-R(36);
         b Null oder 1;
         d 1, 2, 3, 4, 5, 6 oder 7;
         n Null, 1, 2, 3, oder 4;
         R(36) (C₃-C₈)-Cycloalkyl, Phenyl, Biphenylyl oder Naphthyl,
            wobei die Aromaten nicht substituiert oder substituiert sind mit 1 - 3 Substituenten aus der Gruppe bestehend aus F, Cl, CF₃, Methyl, Methoxy und NR(37)R(38);
            R(37) und R(38)
               H, (C₁-C₄)-Alkyl oder (C₁-C₄)-Perfluoralkyl;
      R(34) H oder (C₁-C₄)-Alkyl;
R(3), R(4) und R(5)
   unabhängig voneinander wie R(1) oder R(2) definiert;
oder eine Verbindung ausgewählt aus der Gruppe bestehend aus 3-Acetyl-4-hydroxy-benzoylguanidin, 3-Acetyl-5-brom-4-hydroxy-benzoylguanidin-hydrochlorid, 3-Acetyl-6-hydroxy-benzoylguanidin-hydrochlorid, 3-Acetyl-5-bromo-4-hydroxy-benzoylguanidin-hydrochlorid und 3-Acetyl-5-chloro-4-hydroxy-benzoylguanidin-hydrochlorid,
sowie deren pharmazeutisch verträgliche Salze.

Bevorzugt sind Verbindungen der Formel I, in denen bedeuten:
R(1) R(6)-CO oder R(7)R(8)N-CO-;
   R(6) (C₁-C₈)-Alkyl, (C₃-C₈)-Alkenyl oder -CₙH₂ₙ-R(9);
      n Null oder 1;
      R(9) (C₃-C₈)-Cycloalkyl oder Phenyl;
         welches nicht substituiert oder substituiert ist mit 1 - 3 Substituenten aus der Gruppe bestehend aus F, Cl, CF₃, Methyl, Methoxy und NR(10)R(11);
         R(10) und R(11)
            H oder CH₃;
   R(7) H, (C₁-C₄)-Alkyl oder CF₃;
   R(8) H, (C₁-C₄)-Alkyl oder CF₃;
R(2) wie R(1) definiert, oder H, F, Cl, Br, I, CN, (C₁-C₈)-Alkyl, CF₃; (C₃-C₈)-Alkenyl oder -CₙH₂ₙR(15);
   n Null, 1 oder 2;
   R(15) (C₃-C₈)-Cycloalkyl oder Phenyl;
      welches nicht substituiert oder substituiert ist mit 1 - 3 Substituenten aus der Gruppe bestehend aus F, Cl, CF₃, Methyl, Methoxy und NR(16)R(17);
      R(16) und R(17)
         H oder CH₃;
   oder
R(2) (C₁-C₉)-Heteroaryl,
   das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
oder
R(2) -OR(18);
   R(18) -CₐH₂ₐ-(C₁-C₉)-Heteroaryl,
      das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
      a Null oder 1;
   oder
R(2) R(21)-SOₘ;
   m 2;
   R(21) (C₁-C₈)-Alkyl, CF₃, (C₃-C₈)-Alkenyl oder -CₙH₂ₙ-R(24);
      n Null oder 1;
      R(24) (C₃-C₈)-Cycloalkyl oder Phenyl;
         welches nicht substituiert oder substituiert ist mit 1 - 3 Substituenten aus der Gruppe bestehend aus F, Cl, CF₃, Methyl, Methoxy und NR(27)R(28);
         R(27) und R(28)
            H oder CH₃;
   oder
R(2) R(33)X;
   X Sauerstoff, S oder NR(34);
   R(33) (C₁-C₈)-Alkyl, (C₃-C₈)-Alkenyl, (CH₂)_{b}C_{d}F_{2d+1} oder -CₙH₂ₙ-R(36);
      b Null oder 1;
      d 1, 2, 3, 4, 5, 6 oder 7;
      n Null oder 1;
      R(36) (C₃-C₈)-Cycloalkyl oder Phenyl,
         welches nicht substituiert oder substituiert ist mit 1 - 3 Substituenten aus der Gruppe bestehend aus F, Cl, CF₃, Methyl, Methoxy und NR(37)R(38);
         R(37) und R(38)
            H oder CH₃;
   R(34) H oder (C₁-C₄)-Alkyl;
R(3), R(4) und R(5)
   unabhängig voneinander wie R(1) oder R(2) definiert;
oder eine Verbindung ausgewählt aus der Gruppe bestehend aus 3-Acetyl-4-hydroxy-benzoylguanidin, 3-Acetyl-5-brom-4-hydroxy-benzoylguanidin-hydrochlorid, 3-Acetyl-6-hydroxy-benzoylguanidin-hydrochlorid, 3-Acetyl-5-bromo-4-hydroxy-benzoylguanidin-hydrochlorid und 3-Acetyl-5-chloro-4-hydroxy-benzoylguanidin-hydrochlorid,
sowie deren pharmazeutisch verträgliche Salze.

Besonders bevorzugt sind Verbindungen der Formel I, in der bedeuten:
R(1) R(6)-CO oder R(7)R(8)N-CO-;
   R(6) (C₁-C₄)-Alkyl, (C₃-C₈)-Alkenyl oder -CₙH₂ₙ-R(9);
      n Null oder 1;
      R(9) (C₃-C₈)-Cycloalkyl oder Phenyl;
         welches nicht substituiert oder substituiert ist mit 1 - 3 Substituenten aus der Gruppe bestehend aus F, Cl, CF₃, Methyl, Methoxy und NR(10)R(11);
         R(10) und R(11)
            H oder CH₃;
   R(7) H, (C₁-C₄)-Alkyl oder CF₃;
   R(8) H, (C₁-C₄)-Alkyl oder CF₃;
R(2) wie R(1) definiert, oder H, F, Cl, Br, I, CN, (C₁-C₈)-Alkyl, CF₃ oder -CₙH₂ₙR(15);
   n Null, 1 oder 2;
   R(15) (C₃-C₈)-Cycloalkyl oder Phenyl;
      welches nicht substituiert oder substituiert ist mit 1 - 3 Substituenten aus der Gruppe bestehend aus F, Cl, CF₃, Methyl, Methoxy und NR(16)R(17);
      R(16) und R(17)
         H oder CH₃;
   oder
R(2) Chinolyl, Isochinolyl, Pyrrolyl oder Pyridyl,
   die über C oder N verknüpft sind und die unsubstituiert oder substituiert sind mit 1 - 3 Substituenten aus der Gruppe bestehend aus F, Cl, CF₃, CH₃, Methoxy, Hydroxy, Amino, Methylamino und Dimethylamino;
   oder
R(2) -OR(18);
   R(18) Chinolyl, Isochinolyl, Pyrrolyl oder Pyridyl,
      die unsubstituiert oder substituiert sind mit 1 - 3 Substituenten aus der Gruppe bestehend aus F, Cl, CF₃, CH₃, Methoxy, Hydroxy, Amino, Methylamino und Dimethylamino;
   oder
R(2) R(33)X;
   X Sauerstoff, S oder NR(34);
   R(33) (C₁-C₈)-Alkyl, (CH₂)_{b}C_{d}F_{2d+1} oder -CₙH₂ₙ-R(36);
      b Null oder 1;
      d 1, 2, 3, 4, 5, 6 oder 7;
      n Null oder 1;
      R(36) Phenyl,
         welches nicht substituiert oder substituiert ist mit 1 - 3 Substituenten aus der Gruppe bestehend aus F, Cl, CF₃, Methyl, Methoxy und NR(37)R(38);
         R(37) und R(38)
            H oder CH₃;
   R(34) H oder (C₁-C₄)-Alkyl;
R(3) wie R(1) definiert oder H, F, Cl, Br, I, (C₁-C₈)-Alkyl, CF₃ oder -CₙH₂ₙR(15;
   n Null, 1 oder 2;
   R(15) (C₃-C₈)-Cycloalkyl oder Phenyl;
      welches nicht substituiert oder substituiert ist mit 1 - 3 Substituenten aus der Gruppe bestehend aus F, Cl, CF₃, Methyl, Methoxy und NR(16)R(17);
      R(16) und R(17)
         H oder CH₃;
   oder
R(3) Chinolyl, Isochinolyl, Pyrrolyl oder Pyridyl,
   die über C oder N verknüpft sind und die unsubstituiert oder substituiert sind mit 1 - 3 Substituenten aus der Gruppe bestehend aus F, Cl, CF₃, CH₃, Methoxy, Hydroxy, Amino, Methylamino und Dimethylamino;
R(4) und R(5)
   unabhängig voneinander Wasserstoff, NHR(60) oder NR(60)(C₁-C₄)-Alkyl; R(60) H oder (C₁-C₄)-Alkyl;
oder eine Verbindung ausgewählt aus der Gruppe bestehend aus 3-Acetyl-4-hydroxy-benzoylguanidin, 3-Acetyl-5-brom-4-hydroxy-benzoylguanidin-hydrochlorid, 3-Acetyl-6-hydroxy-benzoylguanidin-hydrochlorid, 3-Acetyl-5-bromo-4-hydroxy-benzoylguanidin-hydrochlorid und 3-Acetyl-5-chloro-4-hydroxy-benzoylguanidin-hydrochlorid,
sowie deren pharmazeutisch verträgliche Salze.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(60) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung der Verbindung I, dadurch gekennzeichnet, daß man eine Verbindung der Formel II 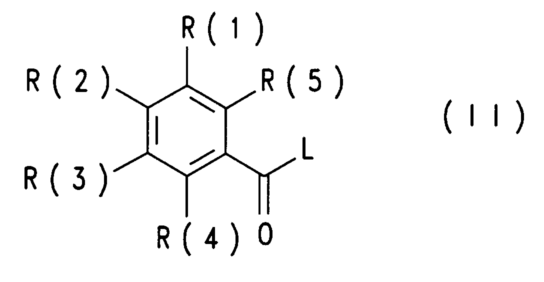 mit Guanidin umsetzt, worin R(1) bis R(5) die angegebene Bedeutung besitzen und L für eine leicht nucleophil substituierbare Fluchtgruppe steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure- derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L= 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyl uronium-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.
Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. -zink-Verbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 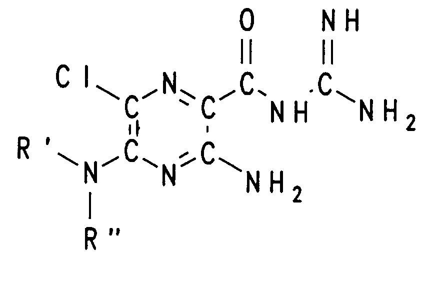
Amilorid: R',R'' = H
Dimethylamilorid: R',R'' = CH₃
Ethylisopropylamilorid: R' = C₂H₅, R'' = CH(CH₃)₂

Darüber hinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034) werden Benzoylguanidine vorgeschlagen, in welchen aber die Substituenten nicht die nach der vorliegenden Erfindung beanspruchten Bedeutungen haben.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- EI: electron impact
- DCI: Desorption-Chemical Ionisation
- RT: Raumtemperatur
- EE: Ethylacetat (EtOAc)
- mp: Schmelzpunkt
- HEP: n-Heptan
- ES: Elektronenspray
- FAB: Fast Atom Bombardment
- CH₂Cl₂: Dichlormethan
- THF: Tetrahydrofuran
- eq.: Äquivalent
- mol%: Molprozente
- (dppf): 1,1-Bis(diphenylphosphino)ferrocen
- AIBN: Azobisisobutyronitril

### Experimenteller Teil

### Allgemeine Vorschrift zur Herstellung von Benzoyl-guanidinen (I)

### Variante A: aus Benzoesäuren (II, L = OH)

1.0 eq. des Benzoesäurederivats der Formel II löst bzw. suspendiert man in wasserfreiem THF ( 5 ml/mmol ) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCl auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: 3-Acetyl-4-hydroxy-benzoylguanidin

1 a) 4-Acetoxy-benzoesäure wurde durch Fries-Verschiebung mit 3 eq. AlCl₃ bei 140°C in die 3-Acetyl-4-hydroxy-benzoesäure überführt.
1 b) 1.0 eq. 3-Acetyl-4-hydroxy-benzoesäure wurden gemäß Variante A mit 1.1 eq. Carbonyldiimidazol und 5 eq. Guanidin umgesetzt.
Farblose Kristalle, mp 218 - 221 °C.

### Beispiel 2: 3-Acetyl-4-methoxy-benzoylguanidin-hydrochlorid

2 a) 1.0 eq. 3-Acetyl-4-hydroxy-benzoesäure [Darstellung siehe 1 a)] wurden mit jeweils 2.2 eq. K₂CO₃ und Methyliodid in absoluten Aceton zum 3-Acetyl-4-methoxy-benzoesäuremethylester umgesetzt.
Farblose Kristalle, mp 54 - 58°C.
2 b) Der Ester aus 2 a) wurde gemäß Variante B mit Guanidin umgesetzt.
Farblose Kristalle, mp 156 - 162°C.
Das Hydrochlorid wurde gemäß Variante A dargestellt.
Farblose Kristalle, mp 206 - 212°C.

### Beispiel 3: 3-Acetyl-4-isopropoxy-benzoylguanidin-hydrochlorid

3 a) 1.0 eq. 3-Acetyl-4-hydroxy-benzoesäure wurden mit jeweils 2.2 eq. K₂CO₃ und Isopropylbromid in absoluten DMF zum 3-Acetyl-4-isopropoxybenzoesäureisopropylester umgesetzt.
Gelbliches Öl, MS (ES): 265 (M+1).
3 b) Der Ester aus 3 a) wurde gemäß Variante B mit Guanidin umgesetzt und ins Hydrochlorid überführt.
Farblose Kristalle, mp 158 - 170°C.

### Beispiel 4: 3-Acetyl-4-benzyloxy-benzoylguanidin-hydrochlorid

4 a) 1.0 eq. 3-Acetyl-4-hydroxy-benzoesäuremethylester wurden mit jeweils 1.1 eq. K₂CO₃ und Benzylbromid in absoluten DMF zum 3-Acetyl-4-benzyloxybenzoesäuremethylester umgesetzt.
Farblose Kristalle, mp 68 - 72°C.
4 b) Der Ester aus 4 a) wurde gemäß Variante B mit Guanidin umgesetzt und als Hydrochlorid isoliert.
Farblose Kristalle, mp 175 - 182°C.

### Beispiel 5: 3-Acetyl-5-brom-4-hydroxy-benzoylguanidin-hydrochorid

Der literaturbekannte 3-Acetyl-5-brom-4-hydroxy-benzoesäuremethylester wurde gemäß Variante B ins Benzoylguanidin überführt und als Hydrochlorid isoliert.
Farblose Kristalle, mp 230°C (unter Zersetzung).

### Beispiel 6: 3-Acetyl-benzoylguanidin-hydrochlorid

1.0 eq. 3-Acetyl-benzoesäure wurden gemäß Variante A mit 1.1 eq. Carbonyldiimidazol und 5 eq. Guanidin umgesetzt und als Hydrochlorid isoliert.
Farblose Kristalle, mp 202 - 204°C.

### Beispiel 7: 3-Benzoyl-benzoylguanidin-hydrochlorid

1.0 eq. 3-Benzoyl-benzoesäure wurden gemäß Variante A mit 1.1 eq. Carbonyldiimidazol und 5 eq. Guanidin umgesetzt und als Hydrochlorid isoliert.
Farblose Kristalle, mp 150 - 154°C.

### Beispiel 8: 3-Acetyl-4-isopropyl-benzoylguanidin-hydrochlorid

8 a) 3-Acetyl-4-hydroxy-benzoesäuremethylester wurde in CH₂Cl₂ mit Pyridin und Trifluormethansulfonsäureanhydrid in das 4-Triflat überführt.
Gelbliches Öl, MS (ES): 327 (M+1).
8 b) 1.0 eq. des Triflats aus 8 a) wurde in THF gelöst und mit 3 mol% PdCl₂(dppf) und 4 mol% Kupfer-(I)-iodid versetzt. Zu dieser Suspension gab man 1.5 eq. Isopropyl-zinkchlorid (dargestellt aus dem Grignard-Reagenz und Zinkchlorid) und rührte bei RT. Standardaufarbeitung mit 1 N HCl ergab nach säulenchromatographischer Aufarbeitung 3-Acetyl-4-isopropylbenzoesäuremethylester.
Öl, MS (ES): 221 (M+1).
8 c) Das Benzoesäuremethylesterderivat aus 8 b) wurde unter Standardbedingungen (MeOH/NaOH) verseift und die resultierende Benzoesäure gemäß Variante A ins Guanidid überführt und als Hydrochlorid isoliert.
Farblose Kristalle, mp 107 - 118°C.

### Beispiel 9: 3-Acetyl-4-isobutyl-benzoylguanidin-hydrochlorid

Wurde in Analogie zu Beispiel 8 dargestellt.
Farblose Kristalle, mp 124 - 130°C.

### Beispiel 10: 3-Acetyl-4-cyclopentyl-benzoylguanidin-hydrochlorid

Wurde in Analogie zu Beispiel 8 dargestellt.
Farblose Kristalle, mp 148 - 154°C.

### Beispiel 11: 3-Acetyl-4-(2,2-dimethylpropyl)-benzoylguanidin-hydrochlorid

Wurde in Analogie zu Beispiel 8 dargestellt.
Farblose Kristalle, mp 138 - 148°C.

### Beispiel 12: 3-Acetyl-4-fluor-benzoylguanidin-hydrochlorid

12 a) 3-Bromo-4-fluoro-benzoesäuremethylester wird gemäß einer literaturbekannten Vorschrift (Ph₃P)₂PdCl₂ katalysiert mit Trimethylsilylacetylen zum 4-Fluoro-3-(2-trimethylsilylethinyl)-benzoesäuremethylester umgesetzt.
MS (ES): 251 (M+1).
12 b) Das Produkt aus 12 a) wird in 90%iger Essigsäure unter Zusatz von konzentrierter Schwefelsäure mit Quecksilberacetat zum 3-Acetyl-4-fluorobenzoesäuremethylester umgesetzt.
MS (ES): 198 (M+1).
12 c) Das Benzoesäuremethylesterderivat aus 12 b) wurde unter Standardbedingungen (MeOH/NaOH) verseift, die resultierende Benzoesäure gemäß Variante A ins Guanidid überführt und als Hydrochlorid isoliert.
Farblose Kristalle, mp 178 - 185°C.

### Beispiel 13: 3-Acetyl-4-imidazolyl-benzoylguanidin-hydrochlorid

1.0 eq. 3-Acetyl-4-fluor-benzoylguanidin-hydrochlorid wurde in DMF unter Zusatz von 1.1 eq. K₂CO₃ mit 2 eq. Imidazol zum 4-Imidazolyl-derivat umgesetzt und als Hydrochlorid isoliert.
Farblose Kristalle, mp >240°C.

### Beispiel 14: 3-Acetyl-4-(2,4-difluorphenoxy)-benzoylguanidin-hydrochlorid

14 a) 1.0 eq. 3-Acetyl-4-fluoro-benzoesäuremethylester [Darstellung siehe Beispiel 12 b)] wurde in DMF unter Zusatz von 1.1 eq. K₂CO₃ und 1.3 eq. 2,4-Difluorophenol zum 3-Acetyl-4-(2,4-difluorophenol)-benzoesäuremethylester umgesetzt.
Farblose Kristalle, mp 85 - 87°C.
14 b) Das Benzoesäuremethylesterderivat aus 14 a) wurde unter Standardbedingungen (MeOH/NaOH) verseift, die resultierende Benzoesäure gemäß Variante A ins Guanidid überführt und als Hydrochlorid isoliert.
Farblose Kristalle, mp 188 - 196°C.

### Beispiel 15: 3-Acetyl-4-(4-fluorphenoxy)-benzoylguanidin-hydrochlorid

Wurde in Analogie zu Beispiel 14 dargestellt.
Farblose Kristalle, mp 200 - 210°C.

### Beispiel 16: 3-Acetyl-4-[2-(6-methyl)pyridyl-methoxy]-benzoylguanidinhydrochlorid

16 a) 1 eq. 3-Acetyl-4-hydroxy-benzoesäuremethylester, 1.2 eq. 2,6-Dimethylpyridin und 0.2 eq. DMAP wurden in CH₂Cl₂ bei -30°C mit 1.2 eq. Trifluormethan-sulfonsäureanhydrid versetzt und 2 h bei RT gerührt. Zugabe von ges. NaHCO₃-Lösung, dreimaliges Ausschütteln mit EE, Trocknen über MgSO₄ und Einengen im Vakuum ergab als Rohprodukt ein gelbes Öl. Dieses wurde in DMF aufgenommen, mit 1 eq. 5-Hydroxy-chinolin und K₂CO₃ versetzt und auf 120°C erwärmt. Nach Aufarbeitung mit ges. NaHCO₃-Lösung/EE wurde säulenchromatographisch gereinigt. Man isolierte 3-Acetyl-4-[2-(6-methyl)pyridylmethoxy]-benzoesäuremethylester.
MS (ES): 300 (M+1).
16 b) Das Benzoesäureester-derivat aus 16 a) wurde gemäß Variante A ins Guanidid überführt.
MS (ES): 327 (M+1).

### Beispiel 17: 3-Acetyl-5-methylsulfonyl-benzoylguanidin-hydrochlorid

17 a) 3-Bromo-benzoesäure wurde durch Behandeln mit Chlorsulfonsäure in die entsprechende 3-Chlorsulfonsäure überführt. Anschließende Hydrolyse, Überführung ins Dinatriumsalz und Dimethylierung mit Methyliodid in DMF ergab 5-Bromo-3-methylsulfonyl-benzoesäuremethylester.
Kristalle, mp 82 - 85°C.
17 b) Das Benzoesäureester-derivat aus 17 a) wurde in Analogie zu Beispiel 12 a) und 12 b) in den 3-Acetyl-5-methylsulfonyl-benzoesäuremethylester überführt.
Farblose Kristalle, mp 106 - 108°C.
17 c) Der Ester aus 17 b) wurde gemäß Variante B ins Guanidid überführt und anschließend als Hydrochlorid isoliert.
Farblose Kristalle, mp 221 - 223°C.

### Beispiel 18: 3-Acetyl-6-hydroxy-benzoylguanidin-hydrochlorid

1.0 eq. 3-Acetyl-6-hydroxy-benzoesäure wurden gemäß Variante A mit 1.1 eq. Carbonyldiimidazol und 5 eq. Guanidin umgesetzt und als Hydrochlorid isoliert.
Farblose Kristalle, mp 170 - 180°C.
Beispiel 19: 3-Acetyl-5-bromo-4-hydroxy-benzoylguanidin-hydrochlorid
19 a) 3-Acetyl-4-hydroxybenzoesäuremethylester wurde in Chlorbenzol mit 1.1 eq. N-Bromsuccinimid und einer Spur AIBN zum 3-Acetyl-5-bromo-4-hydroxybenzoe-säure-methylester umgesetzt.
Farblose Kristalle, mp 106 - 108°C.
19 b) Der Ester aus 19 a) wurde gemäß Variante B ins Guanidid überführt und als Hydrochlorid isoliert.
Kristalle, mp 230°C unter Zersetzung.

### Beispiel 20: 3-Acetyl-5-chloro-4-hydroxy-benzoylguanidin-hydrochlorid

20 a) 1 eq. 4-Acetoxy-3-chloro-benzoesäure wurde mit 3 eq. AlCl₃ vermischt und 1 h bei 140°C erhitzt. Aufarbeitung mit 2 N HCl ergab 3-Acetyl-5-chloro-4-hydroxy-benzoesäure.
Gelbe Kristalle, mp. 226 - 234°C.
20 b) Die Benzoesäure aus 20 a) wurde gemäß Variante A ins Guanidid überführt und als Hydrochlorid isoliert.
Gelbliche Kristalle, mp. 198 - 203°C.

### Beispiel 21: 3-Acetyl-5-bromo-4-methoxy-benzoylguanidin-hydrochlorid

21 a) 1 eq. 3-Acetyl-5-bromo-4-hydroxy-benzoesäuremethylester (Darstellung siehe Bei-spiel 19) wurden in Aceton mit 2.0 eq. K₂CO₃ und 2.1 eq. Methyliodid zum 3-Acetyl-5-bromo-4-methoxy-benzoesäuremethylester umgesetzt.
Farbloses Öl, MS (ES): 288 (M+1).
21 b) Der 4-Methoxy-benzoesäureester aus 21 a) wurde unter Standardbedingungen (MeOH/NaOH) verseift, das resultierende Benzoesäurederivat gemäß Variante A ins Guanidid überführt und als Hydrochlorid isoliert.
Farblose Kristalle, mp 175 - 178°C.

### Beispiel 22: 3-Acetyl-5-bromo-4-isopropoxy-benzoylguanidin-hydrochlorid

Wurde in Analogie zu Beispiel 21 dargestellt.
Farblose Kristalle, mp 158 - 170°C.

### Beispiel 23: 3-Acetyl-5-bromo-4-benzyloxy-benzoylguanidin-hydrochlorid

Wurde in Analogie zu Beispiel 21 dargestellt.
Farblose Kristalle, mp 175 - 182°C.

### Beispiel 24: 3-Acetyl-5-cyclopentyl-4-methoxy-benzoylguanidin-hydrochlorid

24 a) 3-Acetyl-5-bromo-4-methoxy-benzoesäuremethylester wurde gemäß Beispiel 8 b) PdCl₂(dppf) vermittelt mit Cyclopentylzinkchlorid umgesetzt (Br⁻anstelle von CF₃SO₃⁻ als Abgangsgruppe).
Gelbes Öl, MS (ES): 277 (M+1).
24 b) Das Produkt aus 24 a) wurde unter Standardbedingungen (MeOH/NaOH) verseift, das resultierende Benzoesäurederivat gemäß Variante A ins Guanidid überführt und als Hydrochlorid isoliert.
Farbloses Öl, MS (ES): 304 (M+1).

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀ mol/l |
| 1 | 2,0 x 10⁻⁶ |
| 2 | 1,0 x 10⁻⁶ |
| 3 | 1,1 x 10⁻⁶ |
| 4 | 1,2 x 10⁻⁶ |

## Patentansprüche

1. Benzoylguanidine der Formel I 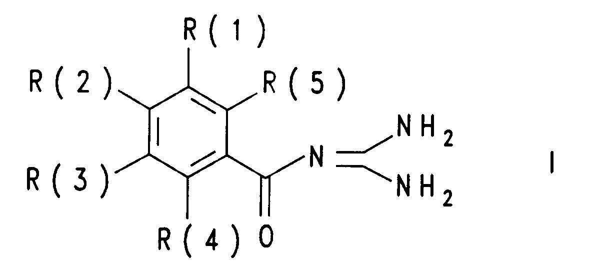 worin bedeuten:
R(1) R(6)-CO oder R(7)R(8)N-CO-;
R(6) (C₁-C₈)-Alkyl, (C₁-C₈)-Perfluoralkyl, (C₃-C₈)-Alkenyl oder -CₙH₂ₙ-R(9);
n Null, 1, 2, 3 oder 4;
R(9) (C₃-C₈)-Cycloalkyl, Phenyl, Biphenylyl oder Naphthyl, wobei die Aromaten nicht substituiert oder substituiert sind mit 1 - 3 Substituenten aus der Gruppe bestehend aus F, Cl, CF₃, Methyl, Methoxy und NR(10)R(11); R(10) und R(11)
H, (C₁-C₄)-Alkyl oder (C₁-C₄)-Perfluoralkyl;
R(7) H, (C₁-C₄)-Alkyl oder CF₃;
R(8) H, (C₁-C₄)-Alkyl oder CF₃;
R(2) wie R(1) definiert, oder H, F, Cl, Br, I, CN, (C₁-C₈)-Alkyl, (C₁-C₈)-Perfluoralkyl, (C₃-C₈)-Alkenyl oder -CₙH₂ₙR(15);
n Null, 1, 2, 3, 4;
R(15) (C₃-C₈)-Cycloalkyl, Phenyl, Biphenylyl oder Naphthyl,
wobei die Aromaten nicht substituiert oder substituiert sind mit 1 - 3 Substituenten aus der Gruppe bestehend aus F, Cl, CF₃, Methyl, Methoxy und NR(16)R(17);
R(16) und R(17)
H, (C₁-C₄)-Alkyl oder (C₁-C₄)-Perfluoralkyl;
oder
R(2) (C₁-C₉)-Heteroaryl,
das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
oder
R(2) -OR(18);
R(18) -CₐH₂ₐ-(C₁-C₉)-Heteroaryl,
das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
a Null, 1 oder 2;
oder
R(2) R(21)-SOₘ;
m 1 oder 2;
R(21) (C₁-C₈)-Alkyl, (C₁-C₈)-Perfluoralkyl, (C₃-C₈)-Alkenyl oder - CₙH₂ₙ-R(24);
n Null, 1, 2, 3 oder 4;
R(24) (C₃-C₈)-Cycloalkyl, Phenyl, Biphenylyl oder Naphthyl,
wobei die Aromaten nicht substituiert oder substituiert sind mit 1 - 3 Substituenten aus der Gruppe bestehend aus F, Cl, CF₃, Methyl, Methoxy und NR(27)R(28);
R(27) und R(28)
H, (C₁-C₄)-Alkyl oder (C₁-C₄)-Perfluoralkyl;
oder
R(2) R(33)X;
X Sauerstoff, S oder NR(34);
R(33) (C₁-C₈)-Alkyl, (C₃-C₈)-Alkenyl, (CH₂)_{b}C_{d}F_{2d+1} oder -CₙH₂ₙ-R(36);
b Null oder 1;
d 1, 2, 3, 4, 5, 6 oder 7;
n Null, 1, 2, 3, oder 4;
R(36) (C₃-C₈)-Cycloalkyl, Phenyl, Biphenylyl oder Naphthyl,
wobei die Aromaten nicht substituiert oder substituiert sind mit 1 - 3 Substituenten aus der Gruppe bestehend aus F, Cl, CF₃, Methyl, Methoxy und NR(37)R(38);
R(37) und R(38)
H, (C₁ -C₄)-Alkyl oder (C₁-C₄)-Perfluoralkyl;
R(34) H oder (C₁-C₄)-Alkyl;
R(3), R(4) und R(5)
unabhängig voneinander wie R(1) oder R(2) definiert;
oder eine Verbindung ausgewählt aus der Gruppe bestehend aus 3-Acetyl-4-hydroxy-benzoylguanidin, 3-Acetyl-5-brom-4-hydroxy-benzoylguanidinhydrochlorid, 3-Acetyl-6-hydroxy-benzoyguanidin-hydrochlorid, 3-Acetyl-5-bromo-4-hydroxy-benzoylguanidin-hydrochlorid und 3-Acetyl-5-chloro-4-hydroxybenzoylguanidin-hydrochlorid,
sowie deren pharmazeutisch verträgliche Salze.

2. Verbindungen der Formel I nach Anspruch 1, in der bedeuten:
R(1) R(6)-CO oder R(7)R(8)N-CO-;
R(6) (C₁-C₈)-Alkyl, (C₃-C₈)-Alkenyl oder -CₙH₂ₙ-R(9);
n Null oder 1;
R(9) (C₃-C₈)-Cycloalkyl oder Phenyl;
welches nicht substituiert oder substituiert ist mit 1 - 3 Substituenten aus der Gruppe bestehend aus F, Cl, CF₃, Methyl, Methoxy und NR(10)R(11);
R(10) und R(11)
H oder CH₃;
R(7) H, (C₁-C₄)-Alkyl oder CF₃;
R(8) H, (C₁-C₄)-Alkyl oder CF₃;
R(2) wie R(1) definiert, oder H, F, Cl, Br, I, CN, (C₁-C₈)-Alkyl, CF₃; (C₃-C₈)-Alkenyl oder -CₙH₂ₙR(15);
n Null, 1 oder 2;
R(15) (C₃-C₈)-Cycloalkyl oder Phenyl;
welches nicht substituiert oder substituiert ist mit 1 - 3 Substituenten aus der Gruppe bestehend aus F, Cl, CF₃, Methyl, Methoxy und NR(16)R(17);
R(16) und R(17)
H oder CH₃;
oder
R(2) (C₁-C₉)-Heteroaryl,
das über C oder N verknüpft ist und das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
oder
R(2) -OR(18);
R(18) -CₐH₂ₐ-(C₁-C₉)-Heteroaryl,
das unsubstituiert oder substituiert ist mit 1 - 3 Substituenten aus der Gruppe bestehend aus F, Cl, CF₃, CH₃, Methoxy, Hydroxy, Amino, Methylamino und Dimethylamino;
a Null oder 1;
oder
R(2) R(21)-SOₘ;
m 2;
R(21) (C₁-C₈)-Alkyl, CF₃, (C₃-C₈)-Alkenyl oder -CₙH₂ₙ-R(24);
n Null oder 1;
R(24) (C₃-C₈)-Cycloalkyl oder Phenyl;
welches nicht substituiert oder substituiert ist mit 1 - 3 Substituenten aus der Gruppe bestehend aus F, Cl, CF₃, Methyl, Methoxy und NR(27)R(28);
R(27) und R(28)
H oder CH₃;
oder
R(2) R(33)X;
X Sauerstoff, S oder NR(34);
R(33) (C₁-C₈)-Alkyl, (C₃-C₈)-Alkenyl, (CH₂)_{b}C_{d}F_{2d+1} oder -CₙH₂ₙ-R(36);
b Null oder 1;
d 1, 2, 3, 4, 5, 6 oder 7;
n Null oder 1;
R(36) (C₃-C₈)-Cycloalkyl oder Phenyl,
welches nicht substituiert oder substituiert ist mit 1 - 3 Substituenten aus der Gruppe bestehend aus F, Cl, CF₃, Methyl, Methoxy und NR(37)R(38);
R(37) und R(38)
H oder CH₃;
R(34) H oder (C₁-C₄)-Alkyl;
R(3), R(4) und R(5)
unabhängig voneinander wie R(1) oder R(2) definiert;
oder eine Verbindung ausgewählt aus der Gruppe bestehend aus 3-Acetyl-4-hydroxy-benzoylguanidin, 3-Acetyl-5-brom-4-hydroxy-benzoylguanidinhydrochlorid, 3-Acetyl-6-hydroxy-benzoylguanidin-hydrochlorid, 3-Acetyl-5-bromo-4-hydroxy-benzoylguanidin-hydrochlorid und 3-Acetyl-5-chloro-4-hydroxybenzoylguanidin-hydrochlorid,
sowie deren pharmazeutisch verträgliche Salze.

3. Verbindungen der Formel I nach Anspruch 1, in der bedeuten:
R(1) R(6)-CO oder R(7)R(8)N-CO-;
R(6) (C₁-C₄)-Alkyl, (C₃-C₈)-Alkenyl oder -CₙH₂ₙ-R(9);
n Null oder 1;
R(9) (C₃-C₈)-Cycloalkyl oder Phenyl;
welches nicht substituiert oder substituiert ist mit 1 - 3 Substituenten aus der Gruppe bestehend aus F, Cl, CF₃, Methyl, Methoxy und NR(10)R(11);
R(10) und R(11)
H oder CH₃;
R(7) H, (C₁-C₄)-Alkyl oder CF₃;
R(8) H, (C₁-C₄)-Alkyl oder CF₃;
R(2) wie R(1) definiert, oder H, F, Cl, Br, I, CN, (C₁-C₈)-Alkyl, CF₃ oder -CₙH₂ₙR(15);
n Null, 1 oder 2;
R(15) (C₃-C₈)-Cycloalkyl oder Phenyl;
welches nicht substituiert oder substituiert ist mit 1 - 3 Substituenten aus der Gruppe bestehend aus F, Cl, CF₃, Methyl, Methoxy und NR(16)R(17);
R(16) und R(17)
H oder CH₃;
oder
R(2) Chinolyl, Isochinolyl, Pyrrolyl oder Pyridyl,
die über C oder N verknüpft sind und die unsubstituiert oder substituiert sind mit 1 - 3 Substituenten aus der Gruppe bestehend aus F, Cl, CF₃, CH₃, Methoxy, Hydroxy, Amino, Methylamino und Dimethylamino;
oder
R(2) -OR(18);
R(18) Chinolyl, Isochinolyl, Pyrrolyl oder Pyridyl,
die unsubstituiert oder substituiert sind mit 1 - 3 Substituenten aus der Gruppe bestehend aus F, Cl, CF₃, CH₃, Methoxy, Hydroxy, Amino, Methylamino und Dimethylamino;
oder
R(2) R(33)X;
X Sauerstoff, S oder NR(34);
R(33) (C₁-C₈)-Alkyl, (CH₂)_{b}C_{d}F_{2d+1} oder -CₙH₂ₙ-R(36);
b Null oder 1;
d 1, 2, 3, 4, 5, 6 oder 7;
n Null oder 1;
R(36) Phenyl,
welches nicht substituiert oder substituiert ist mit 1 - 3 Substituenten aus der Gruppe bestehend aus F, Cl, CF₃, Methyl, Methoxy und NR(37)R(38);
R(37) und R(38)
H oder CH₃;
R(34) H oder (C₁-C₄)-Alkyl;
R(3) wie R(1) definiert oder H, F, Cl, Br, I, (C₁-C₈)-Alkyl, CF₃ oder -CₙH₂ₙR(15;
n Null, 1 oder 2;
R(15) (C₃-C₈)-Cycloalkyl oder Phenyl;
welches nicht substituiert oder substituiert ist mit 1 - 3 Substituenten aus der Gruppe bestehend aus F, Cl, CF₃, Methyl, Methoxy und NR(16)R(17);
R(16) und R(17)
H oder CH₃;
oder
R(3) Chinolyl, Isochinolyl, Pyrrolyl oder Pyridyl,
die über C oder N verknüpft sind und die unsubstituiert oder substituiert sind mit 1 - 3 Substituenten aus der Gruppe bestehend aus F, Cl, CF₃, CH₃, Methoxy, Hydroxy, Amino, Methylamino und Dimethylamino;
R(4) und R(5)
unabhängig voneinander Wasserstoff, NHR(60) oder NR(60)(C₁-C₄)-Alkyl;
R(60) H oder (C₁-C₄)-Alkyl;
oder eine Verbindung ausgewählt aus der Gruppe bestehend aus 3-Acetyl-4-hydroxy-benzoylguanidin, 3-Acetyl-5-brom-4-hydroxy-benzoylguanidin-hydrochlorid, 3-Acetyl-6-hydroxy-benzoylguanidin-hydrochlorid, 3-Acetyl-5-bromo-4-hydroxy-benzoylguanidin-hydrochlorid und 3-Acetyl-5-chloro-4-hydroxy-benzoylguanidin-hydrochlorid,
sowie deren pharmazeutisch verträgliche Salze.

4. Verfahren zur Herstellung einer Verbindung I nach Anspruch 1, dadurch gekennzeichnet, daß man eine Verbindung der Formel II 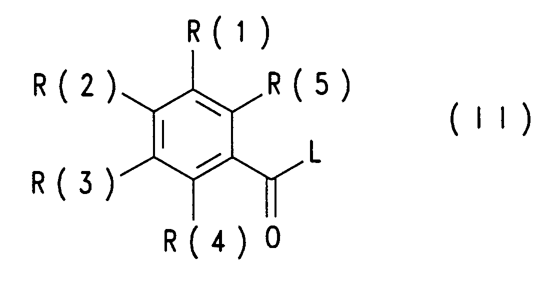 mit Guanidin umsetzt, worin R(1) bis R(5) die in Anspruch 1 angegebene Bedeutung besitzen und L für eine leicht nucleophil substituierbare Fluchtgruppe steht.

5. Verwendung einer Verbindung I nach Anspruch 1 zur Herstellung eines Medikaments zu Behandlung von Arrhythmien.

6. Verwendung einer Verbindung I nach Anspruch 1 zur Herstellung eines Medikaments zur Behandlung oder Prophylaxe des Herzinfarkts.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung von Krankheiten, bei denen die Zellproliferation eine primäre oder sekundäre Ursache darstellt.

15. Heilmittel, enthaltend eine wirksame Menge einer Verbindung I nach Anspruch 1.

## Claims

1. A benzoylguanidine of the formula I 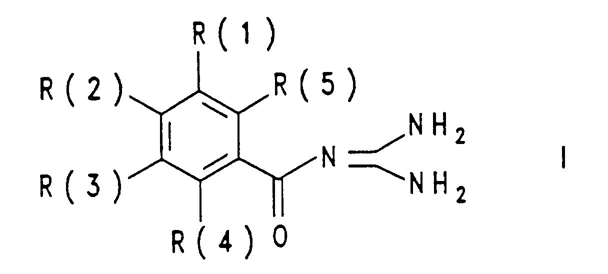 in which:
R(1) is R(6)-CO or R(7)R(8)N-CO-;
R(6) is (C₁-C₈)-alkyl, (C₁-C₈)-perfluoroalkyl,
(C₃-C₈)-alkenyl or -CₙH₂ₙ-R(9);
n is zero, 1, 2, 3 or 4;
R(9) is (C₃-C₈)-cycloalkyl, phenyl, biphenylyl or naphthyl,
where the aromatic radicals are unsubstituted or substituted by 1 - 3 substituents selected from the group comprising F, Cl, CF₃, methyl, methoxy and NR(10)R(11); R(10) and R(11)
are H, (C₁-C₄)-alkyl or (C₁-C₄)-perfluoroalkyl;
R(7) is H, (C₁-C₄)-alkyl or CF₃;
R(8) is H, (C₁-C₄)-alkyl or CF₃;
R(2) is defined as R(1) or is H, F, Cl, Br, I, CN, (C₁-C₈)-alkyl, (C₁-C₈)-perfluoroalkyl, (C₃-C₈)-alkenyl or -CₙH₂ₙR(15);
n is zero, 1, 2, 3 or 4;
R(15) is (C₃-C₈)-cycloalkyl, phenyl, biphenylyl or naphthyl,
where the aromatic radicals are unsubstituted or substituted by 1 - 3 substituents selected from the group comprising F, Cl, CF₃, methyl, methoxy and NR(16)R(17);
R(16) and R(17)
are H, (C₁-C₄)-alkyl or (C₁-C₄)-perfluoroalkyl;
or
R(2) is (C₁-C₉)-heteroaryl,
which is linked via C or N and which is unsubstituted or substituted by 1 - 3 substituents selected from the group comprising F, Cl, CF₃, CH₃, methoxy, hydroxyl, amino, methylamino and dimethylamino;
or
R(2) is -OR(18);
R(18) is -CₐH₂ₐ-(C₁-C₉)-heteroaryl,
which is unsubstituted or substituted by 1 - 3 substituents selected from the group comprising F, Cl, CF₃, CH₃, methoxy, hydroxyl, amino, methyl amino and dimethylamino;
a is zero, 1 or 2,
or
R(2) is R(21)-SOₘ;
m is 1 or 2;
R(21) is (C₁-C₈)-alkyl, (C₁-C₈)-perfluoroalkyl, (C₃-C₈)-alkenyl or -CₙH₂ₙ-R(24);
n is zero, 1, 2, 3 or 4;
R(24) is (C₃-C₈)-cycloalkyl, phenyl, biphenylyl or naphthyl,
where the aromatic radicals are
unsubstituted or substituted by 1 - 3 substituents selected from the group comprising F, Cl, CF₃, methyl, methoxy and NR(27)R(28);
R(27) and R(28)
are H, (C₁-C₄)-alkyl or (C₁-C₄)-perfluoroalkyl;
or
R(2) is R(33)X;
X is oxygen, S or NR(34);
R(33) is (C₁-C₈)-alkyl, (C₃-C₈)-alkenyl, (CH₂)_{b}C_{d}F_{2d+1} or -CₙH₂ₙ-R(36);
b is zero or 1;
d is 1, 2, 3, 4, 5, 6 or 7;
n is zero, 1, 2, 3 or 4;
R(36) is (C₃-C₈)-cycloalkyl, phenyl, biphenylyl or naphthyl,
where the aromatic radicals are unsubstituted or substituted by 1 - 3 substituents selected from the group comprising F, Cl, CF₃, methyl, methoxy and NR(37)R(38);
R(37) and R(38)
are H, (C₁-C₄)-alkyl or (C₁-C₄)-perfluoroalkyl;
R(34) is H or (C₁-C₄)-alkyl;
R(3), R(4) and R(5)
are, independently of each other, defined as R(1) or R(2);
or a compound selected from the group comprising 3-acetyl-4-hydroxybenzoylguanidine, 3-acetyl-5-bromo-4-hydroxybenzoylguanidine hydrochloride, 3-acetyl-6-hydroxybenzoylguanidine hydrochloride, 3-acetyl-5-bromo-4-hydroxybenzoylguanidine hydrochloride and 3-acetyl-5-chloro-4-hydroxybenzoylguanidine hydrochloride,
and pharmaceutically tolerated salts thereof.

2. A compound of the formula I as claimed in claim 1, in which:
R(1) is R(6)-CO or R(7)R(8)N-CO-;
R(6) is (C₁-C₈)-alkyl, (C₃-C₈)-alkenyl or -CₙH₂ₙ-R(9);
n is zero or 1;
R(9) is (C₃-C₈)-cycloalkyl or phenyl;
which is unsubstituted or substituted by 1 - 3 substituents selected from the group comprising F, Cl, CF₃, methyl, methoxy and NR(10)R(11);
R(10) and R(11)
are H or CH₃;
R(7) is H, (C₁-C₄)-alkyl or CF₃;
R(8) is H, (C₁-C₄)-alkyl or CF₃;
R(2) is defined as R(1) or is H, F, Cl, Br, I, CN, (C₁-C₈)-alkyl, CF₃; (C₃-C₈)-alkenyl or -CₙH₂ₙR(15);
n is zero, 1 or 2;
R(15) is (C₃-C₈)-cycloalkyl or phenyl;
which is unsubstituted or substituted by 1 - 3 substituents selected from the group comprising F, Cl, CF₃, methyl, methoxy and NR(16)R(17);
R(16) and R(17)
are H or CH₃;
or
R(2) is (C₁-C₉)-heteroaryl,
which is linked via C or N and which is unsubstituted or substituted by 1 - 3 substituents selected from the group comprising F, Cl, CF₃, CH₃, methoxy, hydroxyl, amino, methylamino and dimethylamino;
or
R(2) is -OR(18);
R(18) is -CₐH₂ₐ-(C₁-C₉)-heteroaryl,
which is unsubstituted or substituted by 1 - 3 substituents selected from the group comprising, F, Cl, CF₃, CH₃, methoxy, hydroxyl, amino, methylamino and dimethylamino;
a is zero or 1;
or
R(2) is R(21)-SOₘ;
m is 2;
R(21) is (C₁-C₈)-alkyl, CF₃, (C₃-C₈)-alkenyl or -CₙH₂ₙR(24);
n is zero or 1;
R(24) is (C₃-C₈)-cycloalkyl or phenyl, which is unsubstituted or substituted by 1 - 3 substituents selected from the group comprising F, Cl, CF₃, methyl, methoxy and NR(27)R(28);
R(27) and R(28)
are H or CH₃;
or
R(2) is R(33)X;
X is oxygen, S or NR(34);
R(33) is (C₁-C₈)-alkyl, (C₃-C₈)-alkenyl, (CH₂)_{b}C_{d}F_{2d+1} or -CₙH₂ₙ-R(36);
b is zero or 1;
d is 1, 2, 3, 4, 5, 6 or 7;
n is zero or 1,
R(36) is (C₃-C₈)-cycloalkyl or phenyl,
which is unsubstituted or substituted by 1 - 3 substituents selected from the group comprising F, Cl, CF₃, methyl, methoxy and NR(37)R(38);
R(37) and R(38)
are H or CH₃;
R(34) is H or (C₁-C₄)-alkyl; R(3), R(4) and R(5)
are, independently of each other, defined as R(1) or R(2);
or a compound selected from the group comprising 3-acetyl-4-hydroxybenzoylguanidine, 3-acetyl-5-bromo-4-hydroxybenzoylguanidinehydrochloride, 3-acetyl-6-hydroxybenzoylguanidine hydrochloride, 3-acetyl-5-bromo-4-hydroxybenzoylguanidine hydrochloride and 3-acetyl-5-chloro-4-hydroxybenzoylguanidine hydrochloride,
and pharmaceutically tolerated salts thereof.

3. A compound of the formula I as claimed in claim 1, in which:
R(1) is R(6)-CO or R(7)R(8)N-CO-;
R(6) is (C₁-C₄)-alkyl, (C₃-C₈)-alkenyl or -CₙH₂ₙ-R(9);
n is zero or 1;
R(9) is (C₃-C₈)-cycloalkyl or phenyl;
which is unsubstituted or substituted by 1 - 3 substituents selected from the group comprising F, Cl, CF₃, methyl, methoxy and NR(10)R(11);
R(10) and R(11)
are H or CH₃;
R(7) is H, (C₁-C₄)-alkyl or CF₃;
R(8) is H, (C₁-C₄)-alkyl or CF₃;
R(2) is defined as R(1) or is H, F, Cl, Br, I, CN, (C₁-C₈)-alkyl, CF₃ or -CₙH₂ₙR(15);
n is zero, 1 or 2;
R(15) is (C₃-C₈)-cycloalkyl or phenyl;
which is unsubstituted or substituted by 1 - 3 substituents selected from the group comprising F, Cl, CF₃, methyl, methoxy and NR(16)R(17);
R(16) and R(17)
are H or CH₃;
or
R(2) is quinolyl, isoquinolyl, pyrrolyl or pyridyl, which are linked via C or N and which are unsubstituted or substituted by 1 - 3 substituents selected from the group comprising F, Cl, CF₃, CH₃, methoxy, hydroxyl, amino, methylamino and dimethylamino;
or
R(2) is -OR(18);
R(18) is quinolyl, isoquinolyl, pyrrolyl or pyridyl
which are unsubstituted or substituted by 1 - 3 substituents selected from the group comprising F, Cl, CF₃, CH₃, methoxy, hydroxyl, amino, methylamino and dimethylamino;
or
R(2) is R(33)X;
X is oxygen, S or NR(34);
R(33) is (C₁-C₈)-alkyl, (CH₂)_{b}C_{d}F_{2d+1} or -CₙH₂ₙ-R(36);
b is zero or 1;
d is 1, 2, 3, 4, 5, 6 or 7;
n is zero or 1;
R(36) is phenyl
which is unsubstituted or substituted by 1 - 3 substituents selected from the group comprising F, Cl, CF₃, methyl, methoxy and NR(37)R(38);
R(37) and R(38)
are H or CH₃;
R(34) is H or (C₁-C₄)-alkyl;
R(3) is defined as R(1) or is H, F, Cl, Br, I, (C₁-C₈)-alkyl, CF₃ or -CₙH₂ₙR(15);
n is zero, 1 or 2;
R(15) is (C₃-C₈)-cycloalkyl or phenyl;
which is unsubstituted or substituted by 1 - 3 substituents selected from the group comprising F, Cl, CF₃, methyl, methoxy and NR(16)R(17);
R(16) and R(17)
are H or CH₃;
or
R(3) is quinolyl, isoquinolyl, pyrrolyl or pyridyl,
which are linked via C or N and which are unsubstituted or substituted by 1 - 3 substituents selected from the group comprising F, Cl, CF₃ CH₃, methoxy, hydroxyl, amino, methylamino and dimethylamino;
R(4) and R(5)
are, independently of each other, hydrogen, NHR(60) or NR(60)(C₁-C₄)-alkyl;
R(60) is H or (C₁-C₄)-alkyl;
or a compound selected from the group comprising 3-acetyl-4-hydroxybenzoylguanidine, 3-acetyl-5-bromo-4-hydroxybenzoylguanidine hydrochloride, 3-acetyl-6-hydroxybenzoylguanidine hydrochloride, 3-acetyl-5-bromo-4-hydroxybenzoylguanidine hydrochloride and 3-acetyl-5-chloro-4-hydroxybenzoylguanidine hydrochloride,
and pharmaceutically tolerated salts thereof.

4. A process for preparing a compound I as claimed in claim 1, wherein a compound of the formula II 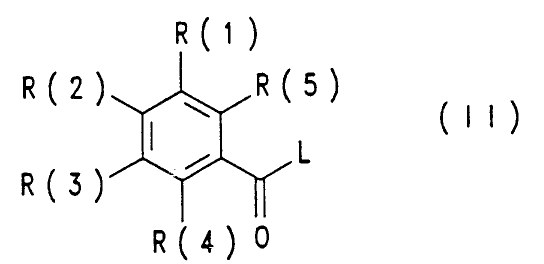 in which R(1) to R(5) have the meaning given in claim 1 and L is a leaving group which can readily be substituted by a nucleophile, is reacted with guanidine.

5. The use of a compound I as claimed in claim 1 for preparing a pharmaceutical for treating arrhythmias.

6. The use of a compound I as claimed in claim 1 for preparing a pharmaceutical for the treatment or prophylaxis of myocardial infarction.

7. The use of a compound I as claimed in claim 1 for preparing a pharmaceutical for the treatment or prophylaxis of angina pectoris.

8. The use of a compound I as claimed in claim 1 for preparing a pharmaceutical for the treatment or prophylaxis of ischemic conditions of the heart.

9. The use of a compound I as claimed in claim 1 for preparing a pharmaceutical for the treatment or prophylaxis of ischemic conditions of the peripheral and central nervous system and of stroke.

10. The use of a compound I as claimed in claim 1 for preparing a pharmaceutical for the treatment or prophylaxis of ischemic conditions of peripheral organs and limbs.

11. The use of a compound I as claimed in claim 1 for preparing a pharmaceutical for treating shock conditions.

12. The use of a compound I as claimed in claim 1 for preparing a pharmaceutical for employment in surgical operations and organ transplants.

13. The use of a compound I as claimed in claim 1 for preparing a pharmaceutical for the preservation and storage of transplants for surgical procedures.

14. The use of a compound I as claimed in claim 1 for preparing a pharmaceutical for treating diseases in which cell proliferation represents a direct or collateral cause.

15. A drug containing an effective quantity of a compound I as claimed in claim 1.

## Revendications

1. Benzoylguanidines de formule I 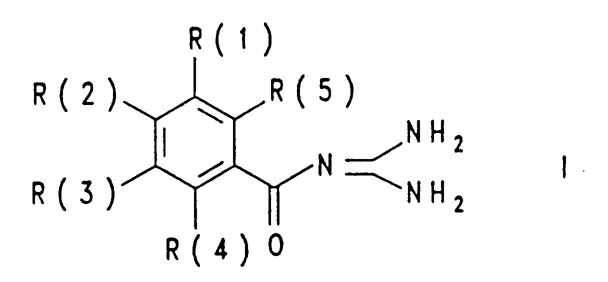 dans laquelle
R(1) représente R(6)CO ou R(7)R(8)N-CO-,
R(6) représente un groupe alkyle en C₁-C₈, perfluoroalkyle en C₁-C₈, alcényle en C₃-C₈ ou -CₙH₂ₙ-R(9),
n est égal à zéro, 1, 2, 3 ou 4,
R(9) représente un groupe cycloalkyle en
C₃-C₈, phényle, biphénylyle ou naphtyle, les radicaux aromatiques n'étant pas substitués ou portant 1 à 3 substituants choisis dans l'ensemble constitué par F, Cl, CF₃, les groupes méthyle, méthoxy et NR(10)R(11),
R(10) et R(11) représentant
H ou un groupe alkyle en C₁-C₄ ou perfluoroalkyle en C₁-C₄;
R(7) représente H ou un groupe alkyle en C₁-C₄ ou CF₃;
R(8) représente H ou un groupe alkyle en C₁-C₄ ou CF₃;
R(2) est défini comme R(1), ou représente H, F, Cl, Br, I, CN, un groupe alkyle en C₁-C₈, perfluoroalkyle en C₁-C₈, alcényle en C₃-C₈ ou -CₙH₂ₙ-R(15),
n est égal à zéro, 1, 2, 3 ou 4,
R(15) représente un groupe cycloalkyle en C₃-C₈, phényle, biphénylyle ou naphtyle, les radicaux aromatiques n'étant pas substitués ou portant 1 à 3 substituants choisis dans l'ensemble constitué par F, Cl, CF₃, les groupes méthyle, méthoxy et NR(16)R(17),
R(16) et R(17) représentant
H ou un groupe alkyle en C₁-C₄ ou perfluoroalkyle en C₁-C₄;
ou
R(2) représente un groupe hétéroaryle en C₁-C₉
qui est relié par C ou N et qui n'est pas substitué ou porte 1 à 3 substituants choisis dans l'ensemble constitué par F, Cl, CF₃, CH₃ et les groupes méthoxy, hydroxyle, amino, méthylamino et diméthylamino;
ou
R(2) représente -OR(18),
R(18) représentant un groupe -CₐH₂ₐ-hétéroaryle(C₁-C₉), qui n'est pas substitué ou porte 1 à 3 substituants choisis dans l'ensemble constitué par F, Cl, CF₃, CH₃ et les groupes méthoxy, hydroxyle, amino, méthylamino et diméthylamino;
a étant zéro, 1 ou 2;
ou
R(2) représente R(21)-SOₘ,
m étant 1 ou 2,
R(21) représentant un groupe alkyle en C₁-C₈, perfluoroalkyle en C₁-C₈, alcényle en C₃-C₈ ou -CₙH₂ₙ-R(24),
n est égal à zéro, 1, 2, 3 ou 4,
R(24) représente un groupe cycloalkyle en C₃-C₈, phényle, biphénylyle ou naphtyle,
les radicaux aromatiques n'étant pas substitués ou portant 1 à 3 substituants choisis dans l'ensemble constitué par F, Cl, CF₃, les groupes méthyle, méthoxy et NR(27)R(28),
R(27) et R(28) représentant un atome d'hydrogène ou un groupe alkyle en C₁-C₄ ou perfluoroalkyle en C₁-C₄;
ou
R(2) représente R(33)X,
X représentant un atome d'oxygène ou de soufre ou un groupe NR(34),
R(33) représentant un groupe
alkyle en C₁-C₈, alcényle en C₃-C₈, (CH₂)_{b}C_{d}F_{2d+1} ou -CₙH₂ₙ-R(36),
b est zéro ou 1,
d est égal à 1, 2, 3, 4, 5, 6 ou 7,
n est égal à zéro, 1, 2, 3 ou 4,
R(36) représente un groupe cycloalkyle en C₃-C₈, phényle, biphénylyle ou naphtyle, les radicaux aromatiques n'étant pas substitués ou portant 1 à 3 substituants choisis dans l'ensemble constitué par F, Cl, CF₃, les groupes méthyle, méthoxy et NR(37)R(38),
R(37) et R(38) représentant un atome d'hydrogène ou un groupe alkyle en C₁-C₄ ou perfluoroalkyle en C₁-C₄;
R(34) représentant H ou un groupe alkyl en (C₁-C₄);
R(3), R(4) et R(5) sont indépendamment les uns des autres définis comme R(1) ou R(2);
ou composé choisi dans l'ensemble constitué par la 3-acétyl-4-hydroxybenzoylguanidine, le chlorhydrate de 3-acétyl-5-bromo-4-hydroxybenzoylguanidine, le chlorhydrate de 3-acétyl-6-hydroxybenzoylguanidine, le chlorhydrate de 3-acétyl-5-bromo-4-hydroxybenzoylguanidine et le chlorhydrate de 3-acétyl-5-chloro-4-hydroxybenzoylguanidine, et leurs sels pharmaceutiquement acceptables.

2. Composés de formule I selon la revendication 1, dans lesquels
R(1) représente R(6)CO ou R(7)R(8)N-CO-,
R(6) représente un groupe alkyle en C₁-C₈, alcényle en C₃-C₈ ou -CₙH₂ₙ-R(9),
n est égal à zéro ou 1,
R(9) représente un groupe cycloalkyle en C₃-C₈ ou phényle
qui n'est pas substitué ou porte 1 à 3 substituants choisis dans l'ensemble constitué par F, Cl, CF₃, les groupes méthyle, méthoxy et
NR(10)R(11),
R(10) et R(11) représentant H ou CH₃;
R(7) représente H ou un groupe alkyle en C₁-C₄ ou CF₃;
R(8) représente H ou un groupe alkyle en C₁-C₄ ou CF₃;
R(2) est défini comme R(1), ou représente H, F, Cl, Br, I, CN, un groupe alkyle en C₁-C₈, CF₃, alcényle en C₃-C₈ ou -CₙH₂ₙ-R(15),
n est égal à zéro, 1 ou 2,
R(15) représente un groupe cycloalkyle en C₃-C₈ ou phényle qui n'est pas substitué ou porte 1 à 3 substituants choisis dans l'ensemble constitué par F, Cl, CF₃, les groupes méthyle, méthoxy et NR(16)R(17),
R(16) et R(17) représentant H ou CH₃;
ou
R(2) représente un groupe hétéroaryle en C₁-C₉ qui est relié par C ou N et qui n'est pas substitué ou porte 1 à 3 substituants choisis dans l'ensemble constitué par F, Cl, CF₃, CH₃ et les groupes méthoxy, hydroxyle, amino, méthylamino et diméthylamino;
ou
R(2) représente -OR(18),
R(18) représentant un groupe -CₐH₂ₐ-hétéroaryle(C₁-C₉), qui n'est pas substitué ou porte 1 à 3 substituants choisis dans l'ensemble constitué par F, Cl, CF₃, CH₃ et les groupes méthoxy, hydroxyle, amino, méthylamino et diméthylamino.
a étant zéro ou 1;
ou
R(2) représente R(21)-SOₘ;
m étant égal à 2;
R(21) représentant un groupe alkyle en C₁-C₈, CF₃, alcényle en C₃-C₈ ou -CₙH₂ₙ-R(24),
n est égal à zéro ou 1,
R(24) représente un groupe cycloalkyle en C₃-C₈ ou phényle
qui n'est pas substitué ou porte 1 à 3 substituants choisis dans l'ensemble constitué par F, Cl, CF₃, les groupes méthyle, méthoxy et NR(27)R(28),
R(27) et R(28) représentant H ou CH₃;
ou
R(2) représente R(33)X
X représentant un atome d'oxygène ou de soufre ou un groupe NR(34),
R(33) représentant un groupe alkyle en C₁-C₈, alcényle en C₃-C₈, (CH₂)_{b}C_{d}F_{2d+1} ou -CₙH₂ₙ-R(36);
b est zéro ou 1,
d est égal à 1, 2, 3, 4, 5, 6 ou 7,
n est égal à zéro ou 1,
R(36) représente un groupe cycloalkyle en C₃-C₈ ou phényle
qui n'est pas substitué ou porte 1 à 3 substituants choisis dans l'ensemble constitué par F, Cl, CF₃, les groupes méthyle, méthoxy et NR(37)R(38),
R(37) et R(38) représentant H ou CH₃
R(34) représentant H ou un groupe alkyl en (C₁-C₄); R(3), R(4) et R(5) sont indépendamment les uns des autres définis comme R(1) ou R(2);
ou composé choisi dans l'ensemble constitué par la 3-acétyl-4-hydroxybenzoylguanidine, le chlorhydrate de 3-acétyl-5-bromo-4-hydroxybenzoylguanidine, le chlorhydrate de 3-acétyl-6-hydroxybenzoylguanidine, le chlorhydrate de 3-acétyl-5-bromo-4-hydroxybenzoylguanidine et le chlorhydrate de 3-acétyl-5-chloro-4-hydroxybenzoylguanidine, et leurs sels pharmaceutiquement acceptables.

3. Composés de formule I selon la revendication 1, dans lesquels
R(1) représente R(6)CO ou R(7)R(8)N-CO-,
R(6) représente un groupe alkyle en C₁-C₈, alcényle en C₃-C₈ ou -CₙH₂ₙ-R(9),
n est égal à zéro ou 1,
R(9) représente un groupe cycloalkyle en C₃-C₈ ou phényle qui n'est pas substitué ou porte 1 à 3 substituants choisis dans l'ensemble constitué par F, Cl, CF₃, les groupes méthyle, méthoxy et NR(10)R(11),
R(10) et R(11) représentant H ou CH₃;
R(7) représente H ou un groupe alkyle en C₁-C₄ ou CF₃;
R(8) représente H ou un groupe alkyle en C₁-C₄ ou CF₃;
R(2) est défini comme R(1), ou représente H, F, Cl, Br, I, CN, un groupe alkyle en C₁-C₈, CF₃ ou -CₙH₂ₙ-R(15),
n est égal à zéro, 1 ou 2,
R(15) représente un groupe cycloalkyle en C₃-C₈ ou phényle qui n'est pas substitué ou porte 1 à 3 substituants choisis dans l'ensemble constitué par F, Cl, CF₃, les groupes méthyle, méthoxy et NR(16)R(17), R(16) et R(17) représentant H ou CH₃;
ou
R(2) représente le groupe quinolyle, isoquinolyle, pyrrolyle ou pyridyle,
qui sont reliés par C ou N et qui ne sont pas substitués ou portent 1 à 3 substituants choisis dans l'ensemble constitué par F, Cl, CF₃, CH₃ et les groupes méthoxy, hydroxyle, amino, méthylamino et diméthylamino;
ou
R(2) représente -OR(18),
R(18) représentant le groupe quinolyle, isoquinolyle, pyrrolyle ou pyridyle,
qui ne sont pas substitués ou portent 1 à 3 substituants choisis dans l'ensemble constitué par F, Cl, CF₃, CH₃ et les groupes méthoxy, hydroxyle, amino, méthylamino et diméthylamino;
ou
R(2) représente R(33)X,
X représentant un atome d'oxygène ou de soufre ou un groupe NR(34);
R(33) représentant un groupe alkyle en C₁-C₈, (CH₂)_{b}C_{d}F_{2d+1} ou -CₙH₂ₙ-R(36);
b est zéro ou 1;
d est égal à 1, 2, 3, 4, 5, 6 ou 7;
n est égal à zéro ou 1;
R(36) représente un groupe phényle
qui n'est pas substitué ou porte 1 à 3 substituants choisis dans l'ensemble constitué par F, Cl, CF₃, les groupes méthyle, méthoxy et NR(37)R(38),
R(37) et R(38) représentant H ou CH₃
R(34) représentant H ou un groupe alkyl en (C₁-C₄);
R(3) est défini comme R(1) ou représente H, F, Cl, Br, I ou un groupe alkyle en C₁-C₈, CF₃ ou -CₙH₂ₙR(15);
n est égal à zéro, 1 ou 2;
R(15) représente un groupe cycloalkyle en C₃-C₈ ou phényle
qui n'est pas substitué ou porte 1 à 3 substituants choisis dans l'ensemble constitué par F, Cl, CF₃, les groupes méthyle, méthoxy et NR(16)R(17),
R(16) et R(17) représentant H ou CH₃;
ou
R(3) représente le groupe quinolyle, isoquinolyle, pyrrolyle ou pyridyle,
qui sont reliés par C ou N et qui ne sont pas substitués ou portent 1 à 3 substituants choisis dans l'ensemble constitué par F, Cl, CF₃, CH₃ et les groupes méthoxy, hydroxyle, amino, méthylamino et diméthylamino;
R(4) et R(5) représentent indépendamment l'un de l'autre un atome d'hydrogène ou un groupe NHR(60) ou NR(60)-alkyle(C₁-C₄);
R(60) représentant H ou un groupe alkyle en C₁-C₄;
ou composé choisi dans l'ensemble constitué par la 3-acétyl-4-hydroxybenzoylguanidine, le chlorhydrate de 3-acétyl-5-bromo-4-hydroxybenzoylguanidine, le chlorhydrate de 3-acétyl-6-hydroxybenzoylguanidine, le chlorhydrate de 3-acétyl-5-bromo-4-hydroxybenzoylguanidine et le chlorhydrate de 3-acétyl-5-chloro-4-hydroxybenzoylguanidine, et leurs sels pharmaceutiquement acceptables.

4. Procédé pour la préparation d'un composé I selon la revendication 1, caractérisé en ce que l'on fait réagir avec de la guanidine un composé de formule II 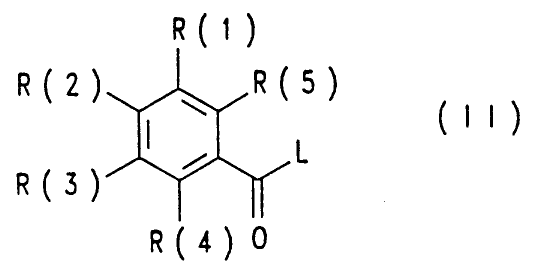 dans laquelle R(1) à R(5) ont les significations données dans la revendication 1 et L représente un groupe partant pouvant être aisément remplacé par substitution nucléophile.

5. Utilisation d'un composé I selon la revendication 1, pour la fabrication d'un médicament destiné au traitement d'arythmies.

6. Utilisation d'un composé I selon la revendication 1, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux périphérique et central et de l'apoplexie cérébrale.

10. Utilisation d'un composé I selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d'organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Médicament, contenant une quantité efficace en un composé de formule I selon la revendication 1.
